**Europäisches Patentamt**

**European Patent Office**

**Office européen des brevets**

(11) Numéro de publication : **0 411 980 B1**

# (12) FASCICULE DE BREVET EUROPEEN

(45) Date de publication du fascicule du brevet :
**16.06.93 Bulletin 93/24**

(51) Int. Cl.$^5$ : **C07H 15/04,** C07H 15/14, C11D 1/66

(21) Numéro de dépôt : **90401991.6**

(22) Date de dépôt : **11.07.90**

(54) **Nouveau procédé de préparation d'alkyl-1-thioglycosides et d'alykl-glycosides nouveaux mélanges d'anomères obtenus par ce procédé et leur application comme détergents non ioniques.**

Demande divisionnaire 92117254.0 déposée le 11/07/90.

(30) Priorité : **31.07.89 FR 8910301**

(43) Date de publication de la demande :
**06.02.91 Bulletin 91/06**

(45) Mention de la délivrance du brevet :
**16.06.93 Bulletin 93/24**

(84) Etats contractants désignés :
**AT BE CH DE DK ES FR GB GR IT LI LU NL SE**

(56) Documents cités :
FR-A- 2 567 891
US-A- 3 314 913
US-A- 3 635 717
CHEM. PHARM. BULL., vol. 33, no. 2, 1985, pages 503-508; S. SAITO et al.: "Synthesis of alkyl-beta-D-thioglucopyranosides, a series of new nonionic detergents"
CHEMICAL ABSTRACTS, vol. 111, no. 7, 14, août 1989, page 811, résumé no. 58266t, Columbus, Ohio, US; & JP-A-63 303 990 (NITTO CHEMICAL INDUSTRY CO., LTD) 12-12-1988

(56) Documents cités :
CHEMICAL ABSTRACTS, vol. 86, no. 21, 23 mai 1977, page 137, résumé no. 151617h, Columbus, Ohio, US; S. LIN et al.: "The interaction of chemically synthesized 21 base pair lac operator with the lac repressor", & ICN-UCLA SYMP. MOL. CELL. BIOL. 1976, 5(Mol. Mech. Control Gene Expression), 143-58
CHEMISTRY LETTERS, 1984, pages 1747-1750, The Chemical Society of Japan; M. HAYASHI et al.: "Simple synthesis of glycosyl fluorides"
CHEMISTRY LETTERS, 1984, pages 1751-1754, The Chemical Society of Japan; W.A. SZAREK et al.: "The synthesis of glycosyl fluorides using pyridinium poly(hydrogen fluoride)"

(73) Titulaire : **ERIDANIA BEGHIN-SAY**
**F-59239 Thumeries (FR)**

(72) Inventeur : **Defaye, Jacques**
**202 chemin du Vercors**
**F-38330 Saint Dismier (FR)**
Inventeur : **Gadelle, Andrée**
**23 Hameau Fleuri, Mont Bonnot**
**F-38330 Saint Dismier (FR)**
Inventeur : **Pedersen, Christian**
**Bredesvinget 18**
**DK-2830 Virum (DK)**

(74) Mandataire : **David, Daniel et al**
**KAYSERBERG Service Propriété Industrielle**
**23 boulevard Georges Clemenceau**
**F-92402 Courbevoie Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 411 980 B1

## Description

La présente invention se rapporte à un nouveau procédé de préparation d'alkyl-1-thioglycosides. Plus particulièrement l'invention concerne un procédé amélioré de préparation d'alkyl-1-thioglycosides à longue chaîne alkyle ou chaîne grasse, par action d'un thiol sur un saccharide choisi parmi les monosaccharides, les di- et oligo-saccharides réducteurs dans le solvant et réactif poly- (fluorure d'hydrogène)- pyridinium.

Les alkyl-1-thioglycosides à chaîne grasse, sont connus pour leurs propriétés d'agents tensio-actifs, et en particulier pour leur utilisation comme surfactants ou détergents. Une caractéristique essentielle de ces familles de dérivés glucidiques est de réunir, simultanément sur la même molécule, des groupes fonctionnels hydrophiles et hydrophobes, ce qui leur permet de former des couches monomoléculaires aux interfaces de solvants non miscibles, et des micelles lorsque leur concentration en solution excède une valeur limitante connue sous le nom de concentration micellaire critique (CMC). Ce comportement physico-chimique est à l'origine des applications extrêmement importantes, aussi bien que diverses, des surfactants comme agents de mouillage, de dispersion, comme agents émulsifiants, moussants et de façon plus générale comme agents de solubilisation. Les surfactants sont également connus pour leurs propriétés thermotropiques et lyotropiques de cristaux liquides.

Les alkyl-1-thioglycosides à chaîne grasse constituent une classe de détergents tout particulièrement intéressante car, étant non ioniques, leur CMC, et donc leurs propriétés, ne sont pas dépendantes de la présence d'un contre-ion. La valeur généralement élevée de leur CMC permet par ailleurs de les éliminer commodément et rapidement par dialyse. Ces caractéristiques ont notamment conduit à privilégier leur utilisation en biochimie, pour la solubilisation, l'extraction et la reconstitution de protéines membranaires, et ce d'autant plus que ces dérivés amphiphiles n'ont généralement pas ou peu d'effets dénaturants sur ces structures biologiques complexes et labiles. Les alkyl-1-thioglycosides à chaîne grasse présentent également l'intérêt, par rapport à leurs analogues alkyl-glycosides, d'être insensibles à l'action de la plupart des enzymes glycosidasiques présents dans les milieux biologiques, comme cela a été montré par des résultats publiés dans Biochem.J., 222 (I984) 829.

Les alkyl-1-thioglycosides sont le plus généralement préparés par action d'un halogénure d'alkyle sur un 1-thioglycose O-acétylé, comme dans le brevet JP 61/7288. Une autre méthode, qui a été récemment préconisée dans Tetrahedron Lett. 29 (1988) 4293, utilise le principe de l'addition radicalaire d'un alkène sur ce même dérivé de 1-thioglycose, en présence d'azobis-(isobutyronitrile). Cependant, de tels procédés sont longs, puisqu'ils nécessitent au minimum quatre étapes et ils utilisent des réactifs coûteux. Par ailleurs, ils conduisent exclusivement à l'anomère 1,2-trans de l'alkyl-1-thio-β-D-glycopyranoside, du fait que l'intermédiaire O-acétyl-1-thioglycopyranose possède lui-même cette même configuration de par le procédé de synthèse qui permet de le préparer. Il résulte de ce fait que seuls ont été décrits, jusqu'à présent, des alkyl-1-thioglycopyranosides à chaîne grasse d'anomérie β-D en série glucopyranose, galactopyranose et xylopyranose, et α-D pour le mannopyranose.

Les alkyl-glycosides à chaîne grasse sont, quant à eux, le plus généralement préparés par des procédés de trans-acétalation en présence d'un catalyseur acide, à partir d'un glycoside d'alcool inférieur, ou encore d'un mélange de l'ose avec un alcool inférieur et un alcool gras, comme cela a été décrit dans les brevets DE-A-1905523 et DE-A-1943689. Un procédé apparenté à la réaction de Koenigs-Knorr et qui fait intervenir l'action d'un alcool à longue chaîne sur un halogénure de glycosyle peracétylé, en présence de sels d'argent, a également été préconisé par Koeltzow et al. dans J. Am. Oil Chem. Soc. 61 (1984) 1651.

Le WO-A-8600906 de la demanderesse décrit un procédé de synthèse d'alkyl-glycosides d'alcools gras par réaction d'un alcool gras avec un aldose, un aldoside ou un polyaldoside dans un solvant et réactif constitué par un mélange dioxanne-fluorure d'hydrogène ou dioxyde de soufre-fluorure d'hydrogène mais outre le fait que les rendements obtenus sont relativement faibles, de l'ordre de 30 %, ce procédé n'est pas utilisable pour la synthèse d'alkyl-oligosaccharides car les conditions qui y sont employées conduisent à la fluorolyse des liaisons interosidiques oxygénées. Des alkyl-glycosides d'alcools inférieurs sont également synthétisés, avec un bon rendement, selon le WO-A-8600906 précité par réaction d'un alcool avec un aldose, un aldoside ou un polyaldoside dans un solvant et réactif constitué par du fluorure d'hydrogène mais la quantité d'alcool utilisé est élevée, le rapport molaire alcool sur équivalent monosaccharidique étant avantageusement de l'ordre de 20 et les alkyl-oligosaccharides inférieurs ne peuvent pas non plus être obtenus pour la raison indiquée précédemment.

Un objet de la présente invention est d'obtenir des alkyl-1-thioglycosides en une étape en partant aussi bien d'un monosaccharide, que d'un di- ou d'un oligo-saccharide.

D'autres objets et avantages de l'invention apparaîtront à la lecture de la description ci-après.

La présente invention répond aux objets précités et fournit un procédé de préparation en une étape d'alkyl-1-thioglycosides à partir d'un saccharide choisi parmi les monosaccharides, les di- et oligo-saccharides réducteurs, caractérisé par le fait que l'on fait réagir ledit saccharide avec un thiol dans le solvant et réactif poly-(fluorure d'hydrogène) - pyridinium. Le produit résultant, alkyl-1-thioglycoside qui se forme rapidement avec un excellent rendement, est ensuite extrait. Le poly(fluorure d'hydrogène) - pyridinium, encore connu sous le nom de réactif fluorure d'hydrogène-pyridine, est un produit commercial, qui peut encore être préparé selon le procédé décrit par Olah et al. dans J. Org. Chem. 44 (1979) 3872.

Le procédé de l'invention permet l'obtention en une étape d'alkyl-1-thioglycosides à chaîne grasse ainsi que celle d'alkyl-1-thioglycosides de thiols inférieurs, en partant aussi bien d'un monosaccharide que d'un di- ou d'un oligo-saccharide réducteur.

Parmi les monosaccharides, on peut utiliser aussi bien les hexoses et les pentoses, et en particulier le D-glucose du fait de son accessibilité, ou encore un amino-désoxy-hexose tel que le 2-acétamido-2-désoxy-D-glucose. En ce qui concerne les di- et oligo-saccharides, et pour les mêmes raisons d'accessibilité, des disaccharides tels que le cellobiose, le lactose et le maltose sont les plus susceptibles d'utilisation, mais des oligo-saccharides de degré de polymérisation supérieur comportant jusqu'à dix motifs monosaccharidiques, et même au-delà, peuvent également être utilisés si les propriétés de détergence attendues du produit final le justifient.

Pour la préparation des alkyl-1-thioglycosides, le rapport entre les réactants thiol et saccharide est, dans le cas d'un monosaccharide, de façon optimale sensiblement équimoléculaire ; dans le cas d'un di- ou oligo-saccharide, on a constaté qu'un excès d'environ 1 mole de thiol par rapport à la stoechiométrie permettait d'améliorer le rendement de la réaction.

La quantité du solvant et réactif poly- (fluorure d'hydrogène) - pyridinium n'est pas critique, elle doit simplement être suffisante pour permettre de travailler en phase homogène. Si l'on a constaté qu'il n'y avait aucun avantage à augmenter la quantité du solvant et réactif poly- (fluorure d'hydrogène) - pyridinium au-delà de la quantité nécessaire à l'homogénéité du milieu réactionnel, il faut toutefois noter qu'aucun inconvénient, hormis l'élévation du prix de revient, ne résulte d'une telle augmentation.

Le procédé de l'invention permet d'obtenir des alkyl-1-thioglycosides à chaîne grasse partant des thiols correspondants. Pour l'obtention d'alkyl-1-thioglycosides à longue chaîne, le réactant thiol sera un thiol à chaîne grasse comportant de façon optimale de 5 à 20 atomes de carbone, mais des thiols à nombre d'atomes de carbone supérieur peuvent également être utilisés. Là encore, on devra tenir compte dans le choix du thiol, de l'équilibre souhaité entre les propriétés d'hydrophilie et de lipophilie du produit final. Le 1-heptanethiol et le 1-octanethiol sont les mercaptans préférés, sans que ce choix puisse être considéré comme limitatif.

La formation de l'alkyl-1-thioglycoside est généralement très rapide et on a constaté qu'un rendement optimal est obtenu pour des temps de réaction de 30 min à 5 h. Le produit brut obtenu dans la réaction, après neutralisation et extraction, est constitué d'un mélange d'anomères 1,2-cis et 1,2-trans des alkyl-1-thioglycopyranosides, dans une proportion respective qui dépend du saccharide utilisé. Pour le D-glucose, cette proportion est d'environ 44 % d'anomère α pour 56 d'anomère β. Une faible proportion du dérivé dithioacétal du monosaccharide utilisé peut être également formée. Il est par exemple voisin de 5 % dans le cas du D-glucose dans les conditions préconisées dans l'Exemple 1 ci-après. Ce contaminant n'est généralement pas gênant dans beaucoup d'applications et, par ailleurs, il est, lorsque cela est souhaité, aisément éliminé par cristallisation.

Les alkyl-1-thioglycosides, obtenus selon le procédé de la présente invention sous forme d'un mélange de leurs anomères α et β, sont nouveaux. Il faut noter à cet égard, comme cela a été indiqué plus haut, que pour un même composé alkyl-1-thioglycoside, l'obtention d'un seul des anomères α mais plus généralement β a été décrite mais jamais les deux.

Le procédé de l'invention s'avère également avantageux pour l'obtention d'alkyl-1-thioglycosides de thiols inférieurs comportant notamment un marquage isotopique sur le résidu aglyconique du fait de la faible quantité de réactant aglyconique mise en jeu.

Les concentrations micellaires critiques, mesurées selon la technique préconisée par Tsuchiya et Saito dans J. Biochem., 96 (1984) 1593, figurent dans le Tableau 1 pour un certain nombre d'alkyl-1-thioglycosides à chaîne grasse préparés par le procédé de l'invention.

EP 0 411 980 B1

## Tableau 1

Concentrations micellaires critiques (CMC) pour quelques alkyl-1-thioglycosides obtenus par le procédé de l'invention.

| Alkyl-1-thioglycoside | CMC (mM) |
|---|---|
| 1-Heptyl-1-thio-$\alpha$,$\beta$-D-glucopyranoside | 4,1 |
| 1-Octyl-1-thio-$\alpha$,$\beta$-D-glucopyranoside | 3,0 |
| 1-Octyl-1-thio-$\alpha$,$\beta$-cellobioside | 0,2 |
| 1-Octyl-1-thio-$\alpha$,$\beta$-lactoside | 0,2 |
| 1-Octyl-1-thio-$\alpha$,$\beta$-maltoside | 0,2 |

A titre de comparaison, la CMC du 1-octyl-1-thio-$\beta$-D-glucopyranoside de l'art antérieur mentionnée dans la littérature est égale à 9,0 mM (Chem. Pharm. Bull., 33 (1985) 503-508, S. Saito et T. Tsuchiya).

Ces valeurs moyennes sont, en particulier, adaptées à l'utilisation de ces détergents pour l'extraction de protéines membranaires. A titre d'exemple non limitatif d'utilisation, quelques alkyl-1-thioglycosides préparés selon le procédé de l'invention, ont été appliqués à l'extraction de protéines membranaires du cortex surrénal bovin. Les cellules de cortex surrénal contiennent en effet des systèmes enzymatiques responsables de la stéroïdogenèse. Ces enzymes sont membranaire dans les mitochondries (cytochromes $P-450_{11\beta}$ et $P-450_{scc}$) et dans les microsomes (cytochrome $P-450_{17\alpha}$). Ils sont usuellement extraits de façon optimale en présence de cholate de sodium. Cependant, la nature ionique de ce détergent conduit à un taux de dénaturation des protéines enzymatiques d'autant plus important que le temps de contact entre celles-ci et le cholate de sodium est plus long.

Ceci a comme conséquence en particulier que ce détergent est difficilement utilisable pour la reconstitution de l'environnement lipidique de ces protéines.

Les rendements comparatifs d'extraction des cytochromes $P-450_{11\beta}$ et $P-450_{scc}$ ainsi que $P-450_{17\alpha}$, rapportés respectivement dans les Tableaux 2 et 3 comparativement à des détergents du commerce, montrent que les alkyl-1-thioglycosides préparés selon le procédé faisant l'objet de l'invention présentent de bonnes propriétés d'extraction et de reconstitution de ces protéines labiles.

On remarque également, à la lecture de ces tableaux, que les rendements d'extraction et les taux respectifs d'inactivation varient de façon sensible selon le détergent utilisé sans que des règles puissent être dégagées dans l'état actuel des connaissances. Il apparaît donc que ces procédés d'extraction de protéines membranaires restent encore largement empiriques et qu'il est, par conséquent, souhaitable de pouvoir disposer du plus grand nombre possible de détergents non ioniques au meilleur coût. C'est en particulier l'un des objectifs de la présente invention.

Le Tableau 2 ci-après donne les rendements comparatifs d'extraction[a] des cytochromes $P-450_{11\beta}$ et $P-450_{scc}$ à partir de mitochondries du cortex surrénal bovin en utilisant quelques détergents obtenus par selon le procédé de la présente invention, ainsi que quelques détergents commerciaux et les taux d'inactivation comparatifs de ces protéines membranaires en fonction du détergent utilisé.

4

Tableau 2

| Détergent | Taux d'extraction | | Taux d'inactivation | |
|---|---|---|---|---|
| | après 1 h | après 2 h | après 1 h | après 2 h |
| 1-Heptyl-1-thio-α,β-D-glucopyranoside | 48 | 47 | 0 | 0 |
| 1-Octyl-1-thio-α,β-D-glucopyranoside | 60 | 72 | 43 | 49 |
| 1-Octyl-1-thio-β-D-glucopyranoside[b] | 18 | 91 | 0 | 48 |
| 1-Octyl-β-D-glucopyranoside[b] | 100 | 100 | 0 | 0 |
| Sodium cholate[c] | 94 | / | 0 | / |
| 1-Heptyl-1-thio-α,β-lactoside | 30 | 50 | 0 | 0 |
| 1-Octyl-1-thio-α,β-lactoside | 55 | 62 | 0 | 0 |
| 1-Octyl-1-thio-α,β-cellobioside | 55 | 20 | 0 | 0 |
| 1-Octyl-1-thio-α,β-maltoside | 48 | 94 | 0 | 21 |

(a)  Les mitochondries du cortex surrénal bovin sont obtenues selon M. Satre et P.V. Vignais (Biochemistry, 13 (1974) 2201). Les protéines membranaires sont extraites par incubation dans un tampon approprié contenant du phosphate de potassium 50 mM pH 7,4, du dithiothréitol 1mM, de l'EDTA 1mM et de la désoxycorticostérone 10 μM. Les concentrations respectives en protéines et en détergents sont de 20 mg et 15 mg/ml. Après incubation, le milieu est centrifugé (100 000 g, 45 mn) et les cytochromes P-450 sont dosés dans le surnageant par enregistrement du spectre différentiel entre la forme réduite $Fe^{2+}$ et le complexe $Fe^{2+}CO$. La dénaturation est estimée par la différence d'absorption mesurée à 450 nm (forme native) et 420 nm (forme dénaturée).

(b)  Produits commerciaux de degré de pureté qualifié "ultra-pur".

(c)  Produit commercial de degré de pureté qualifié "ultra-pur".

Le Tableau 3 donne les rendements comparatifs d'extraction[a] du cytochrome $P-450_{17\alpha}$ à partir de microsomes du cortex surrénal bovin en utilisant quelques détergents obtenus selon le procédé de la présente invention, ainsi que quelques détergents commerciaux et les taux d'inactivation comparatifs de ces protéines membranaires en fonction du détergent utilisé.

Tableau 3

| Détergent | Taux d'extraction | Taux d'inactivation |
|---|---|---|
| 1-Heptyl-1-thio-α,β-D-glucopyranoside | 85 | 0 |
| 1-Octyl-1-thio-α,β-D-glucopyranoside | 93 | 0 |
| 1-Octyl-1-thio-β-D-glucopyranoside[b] | 31 | 0 |
| 1-Octyl-β-D-glucopyranoside[b] | 89 | 20 |
| 1-Heptyl-1-thio-α,β-lactoside | 57 | 0 |
| 1-Octyl-1-thio-α,β-lactoside | 65 | 57 |
| 1-Octyl-1-thio-α,β-cellobioside | 72 | 0 |
| 1-Octyl-1-thio-α,β-maltoside | 58 | 0 |

(a) Les microsomes du cortex surrénal bovin sont obtenus, sous forme d'un culot, après centrifugation (100 000 g) pendant 45 mn du surnageant post-mitochondrial.

Les protéines membranaires sont extraites, comme indiqué précédemment, pendant 30 mn à 4°C. Les concentrations respectives en protéine et en détergent sont de 4 et 15 mg/ml. Après centrifugation, l'activité du cytochrome P-450$_{17\alpha}$ est mesurée dans le surnageant en examinant la transformation de la ($^3$H)-prégnenolone en ($^3$H)-17α-hydroxy-prégnenolone.

(b) Produits commerciaux de degré de pureté qualifié "ultra-pur".

Les Exemples suivants, qui donnent des modes opératoires types pour préparer certains des produits revendiqués, illustrent l'invention sans la limiter.

Exemple 1 : **Préparation du 1-octyl-1-thio-α,β-D-glucopyranoside**

Le D-glucose (1 g, 5,55 mmol), placé dans un récipient en Téflon, est additionné de 1-octanethiol (1 ml, 5,6 mmol) et du réactif commercial poly -(fluorure d'hydrogène)- pyridinium (fluorure d'hydrogène - pyridine 60-70/30 p/p, Fluka, 10 ml). La solution est agitée à température ambiante, à l'aide d'un agitateur magnétique, pendant un temps minimum de 45 mn, mais qui peut être porté à 5 h sans inconvénient. On ajoute ensuite à cette solution de l'éther diéthylique (100 ml), puis on neutralise l'acidité par addition de carbonate de calcium (5 g). Les sels minéraux sont alors éliminés par filtration, puis la solution est concentrée sous pression réduite. Le résidu huileux obtenu est dissous dans l'éther diéthylique (100 ml), puis la solution est lavée par l'eau (50 ml), les eaux de lavage étant elles-mêmes ré-extraites par l'éther diéthylique (2 x 50 ml). Les solutions organiques réunies sont séchées sur du sulfate de sodium puis concentrées. Le 1-octyl-1-thio-$\alpha,\beta$-D-glucopyranoside est obtenu sous forme d'un produit huileux (1,8 g), qui contient au maximum 5 % de D-glucose-di-1-octyl-dithioacétal ainsi qu'un spectre de r.m.n du $^{13}$C permet de l'estimer. Cette même technique spectroscopique permet également d'évaluer à 44 % la proportion de l'anomère $\alpha$ du 1-octyl-1-thio-$\alpha,\beta$-D-glucopyranoside présent, le reste étant constitué par l'anomère $\beta$ (56 %).

Le contaminant D-glucose-di-1-octyl-dithioacétal peut être commodément éliminé par cristallisation dans le méthanol ou l'éthanol, ou bien encore par chromatographie sur colonne de gel de silice (Merck 60, 230-400 mesh) en utilisant un mélange 5:1 (v/v) de chloroforme et de méthanol. Avec ce mélange éluant, le $R_f$ du 1-octyl-1-thio-$\alpha,\beta$-D-glucopyranoside est de 0,15 sur plaque de gel de silice (Merck 60, $F_{254}$) et celui de son anomère $\beta$ de 0,17, ce qui fait que la chromatographie sur colonne peut également être utilisée pour séparer ce mélange anomérique si cela est nécessaire. Dans les utilisations que nous en avons faites néanmoins, c'est le mélange anomérique total qui a été employé. Après élimination du D-glucose-di-1-octyl-dithioacétal, la quantité de 1-octyl-1-thio-$\alpha,\beta$-D-glucopyranoside obtenue sous la forme d'une huile, est de 1,6 g (95 %); $[\alpha]_D$ + 129,2° (c 1,62, DMSO).

Anal. Calc. pour $C_{14}H_{28}O_5S$: C, 54,5; H, 9,0; S, 10,4. Trouvé : C, 54,45; H, 9,59; S, 10,26.

R.m.n.$^{13}$C (50,323 MHz, $CDCl_3$, $\delta$ p.p.m. en référence à $CDCl_3$ à 77,2 p.p.m.): anomère $\alpha$; 86,3 (C-1); 74,5, 72,0, 71,3 (C-3, C-2, C-5); 69,2 (C-4); 60,9 (C-6); 31,8, 30,7, 29,90 (2), 29,3, 22,6 (6 $CH_2$); 30,9 (S-$CH_2$); 14,0 ($CH_3$): anomère $\beta$: 85,9 (C-1); 79,6 (C-3); 77,8 (C-5); 72,7 (C-2); 69,4 (C-4); 61,5 (C-6); 31,8, 30,6, 29,90 (2), 29,1, 22,6 (6 $CH_2$); 30,9 (S-$CH_2$) ; 14,0 ($CH_3$).

## Exemple 2 : **Préparation du 1-octyl-2-acétamido-2-désoxy-1-thio-$\alpha,\beta$-D-glucopyranoside.**

Dans un récipient en Téflon, le 2-acétamido-2-désoxy-D-glucose (1 g, 4,5 mmol) est additionné, à température ambiante, de 1-octanethiol (0,8 ml, 4,5 mmol) et du réactif commercial poly(fluorure d'hydrogène)- pyridinium (10 ml) et la solution est agitée pendant 4 heures. Le 1-octyl-2-acétamido-2-désoxy-1-thio-D-glucopyranoside formé est alors précipité de la solution réactionnelle par addition d'éther diéthylique, séparé par décantation et lavé à plusieurs reprises par l'éther diéthylique. Il est obtenu sous la forme d'une poudre blanche (1,4 g, 87 %) qui contient (r.m.n du $^{13}$C) les anomères $\alpha$ et $\beta$ dans une proportion relative 6:4; $[\alpha]_D$ + 40,3° (c 1,0, DMSO).

Anal. Calc. pour $C_{16}H_{31}O_5NS$: C, 55,8 : H, 9,0 : N, 4,1 : S, 9,0. Trouvé: C, 55,7 : H, 8,89 : N, 4,0 : S, 9,0.

R.m.n. $^{13}$C (50,323 MHz, dimethylsulfoxide-$d_6$, $\delta$ p.p.m. en référence a $(CH_3)_2$ SO à 39,6 p.p.m.) : 84,5 (C-1$\beta$), 83,7 (C-1 $\alpha$) ; 81,4, 75,9, 73,7, 71,2(2), 71,0 (C-3,C-4, C-5) : 61,7, 61,2 (C-6): 55,03, 54,8 (C-2).

## Exemple 3 : **Préparation du 1-heptyl-1-thio-$\alpha,\beta$-D-glucopyranoside.**

Dans un récipient en Téflon, le D-glucose (1 g, 5,55 mmol) est additionné de 1-heptanethiol (0,9 ml, 5,6 mmol) et du réactif poly-(fluorure d'hydrogène) - pyridinium (5-10 ml). La solution est agitée à l'aide d'un agitateur magnétique et traitée ensuite comme dans l'Exemple 1. Après élimination du contaminant D-glucose-di-1-heptyl-dithioacétal, le 1-heptyl-1-thio-$\alpha,\beta$-D-glucopyranoside (1,4 g, 85 %) est obtenu sous forme d'une huile avec une proportion relative 44:56 d'anomères $\alpha,\beta$ (r.m.n. $^{13}$C) : $[\alpha]_D$ + 147,3° (c 1,2, DMSO).

Anal. Calc. pour $C_{13}H_{26}O_5S$: C, 53,1 ; H, 8,8 : S, 10,9 : trouvé: C, 53,1 : H, 9,1 ; S, 10,3.

R.m.n. $^{13}$C (50,323 MHz, $CDCl_3$, $\delta$ p.p.nm. en référence à $CDCl_3$ à 77,2 p.p.m.): anomère $\alpha$ ; 86,8 (C-1); 75,0, 72,3, 71,8, 69,8 (C-2, C-3, C-4, C-5); 61,6 (C-6): anomère $\beta$; 86,4 (C-1); 79,6, 77,8, 72,8, 69,5 (C-2, C-3, C-4, C-5); 61,6 (C-6).

## Exemple 4 : **Préparation des 1-octyl-1-thio-disaccharides dérivés du cellobiose, du lactose et du maltose.**

Dans un récipient en Téflon, le cellobiose, le lactose ou le maltose (1 g, 2,9 mmol) sont additionnés de 1-octanethiol (1 ml, 5,6 mmol) et du réactif commercial poly -(fluorure d'hydrogène)- pyridinium (5 ml). La solution est agitée à l'aide d'un agitateur magnétique, à température ambiante pendant 1 h. De l'éther diéthylique (180-200 ml) est ensuite ajouté, et le précipité obtenu est séparé par décantation. Une nouvelle quantité d'éther diéthylique (100 ml) est encore ajoutée, puis la solution est additionnée de carbonate de calcium jusqu'à réaction neutre. Les sels minéraux sont ensuite éliminés par filtration et le filtrat est concentré sous pression réduite. Le résidu huileux obtenu est dissous dans le méthanol (20 ml). L'addition d'éther à la solution méthanolique permet de précipiter les 1-octyl-1-thiodisaccharides correspondants qui sont alors obtenus par filtration ou encore décantation (1,1 g, 80 %). Les proportions relatives des mélanges anomériques sont calculées à partir des intégrations des valeurs des signaux des C-6 en r.m.n. du $^{13}$C. Ces résultats spectroscopiques sont obtenus à 50,123 MHz dans le diméthylsulfoxide-$d_6$ et les descriptions des spectres qui suivent sont données en $\delta$ p.p.m. en référence à $(CH_3)_2SO$ à 72,0 p.p.m. Les produits sont caractérisés comme suit :

-1-Octyl-1-thio-$\alpha,\beta$-cellobioside: $[\alpha]_D$ + 29,2° (c 1,23, DMSO).

Anal. Calc. pour $C_{20}H_{38}O_{10}S$: C, 51,1; H, 8,1; S, 6,8. Trouvé: C, 51,3; H, 8,23; S, 7,09

R.m.n. $^{13}$C : 103,2 (C-1'): 85,0 (C-1 $\alpha,\beta$); 61,2 (C-6'); 60,7 (C-6 $\beta$): 60,2 (C-6$\alpha$) ; $\alpha/\beta$ 1:1.

-1-Octyl-1-thio-$\alpha,\beta$-lactoside: $[\alpha]_D$ + 46,7° (c 1,5, DMSO),

Anal. Calc. pour $C_{20}H_{38}O_{10}S$ : C, 51,1 : H, 8,1 ; S, 6,8. Trouvé: C, 51,2 ; H, 8,24 ; S, 70.

R.mm.n. $^{13}$C : 103,6 (C-1'); 84,9 (C-1 $\alpha,\beta$); 60,5 (C-6'); 60,7 (C-6 $\beta$), 60,2 (C-6 $\alpha$) ; $\alpha/\beta$ 1:1.

-1-Octyl-1-thio-$\alpha,\beta$-maltoside: $[\alpha]_D$ + 43° (c 1,77, DMSO).

Anal. Calc. pour $C_{20}H_{38}O_{10}S$: C,51,1; H, 8,1; S, 6,8. Trouvé: C, 50,5; H, 7,9; S, 67.

R.m.n. $^{13}$C ; 101,1 (C-1') ; 85,4 (C-1 $\alpha$, $\beta$) ; 61,3 (C-6 $\beta$) ; 61,0 (C-6 $\alpha$, C-6').

## Revendications

1. Procédé de préparation d'alkyl-1-thioglycosides à chaîne grasse à partir d'un saccharide choisi parmi les monosaccharides, les di- et les oligo-saccharides réducteurs, caractérisé par le fait que l'on fait réagir ledit saccharide avec un thiol dans le solvant et réactif poly- (fluorure d'hydrogène) - pyridinium le nombre d'atomes de carbone dudit thiol compris entre 5 et 20.

2. Procédé selon la revendication 1 caractérisé par le fait que ledit saccharide est un monosaccharide choisi parmi les hexoses et les pentoses.

3. Procédé selon la revendication 2 caractérisé par le fait que ledit monosaccharide est le D-glucose.

4. Procédé selon la revendication 2 caractérisé par le fait que ledit monosaccharide est un amino-désoxy-hexose.

5. Procédé selon la revendication 1 caractérisé par le fait que ledit saccharide est un disaccharide choisi parmi le cellobiose, le lactose et le maltose.

6. Procédé de préparation d'alkyl-1-thioglycosides selon la revendication 1 caractérisé par le fait que le rapport molaire entre ledit thiol et ledit saccharide est compris entre environ 1 et 2.

7. Procédé de préparation d'alkyl-1-thioglycosides selon la revendication 6 caractérisé par le fait que ledit rapport molaire entre le thiol et le saccharide est sensiblement égal à 1.

8. Alkyl-1-thioglycosides préparés selon le procédé de la revendication 1 caractérisés par le fait qu'ils se trouvent sous forme de leur mélange d'anomères $\alpha$ et $\beta$ et qu'ils comportent une longue chaîne alkyle ou chaîne grasse.

9. Application des alkyl-1-thioglycosides selon la revendication 8 comme détergents non ioniques, en particulier pour la solubilisation, l'extraction et la reconstitution de protéines membranaires.

## Patentansprüche

1. Verfahren zur Herstellung von Alkyl-1-thioglykosiden mit Fettkette aus einem Saccharid, das unter den Monosacchariden und den reduzierenden Di- und Oligosacchariden ausgewählt ist, dadurch gekennzeichnet, daß man das Saccharid mit einem Thiol in dem Lösungsmittel und Reagenz Poly-(fluorwasserstoff)-pyridin reagieren läßt, wobei die Zahl der C-Atome des Thiols 5 bis 20 beträgt.

2. Verfahren nach Anspruch 1, dadurch gekennzeichnet, daß das Saccharid ein Monosaccharid ist, das unter den Hexosen und Pentosen ausgewählt ist.

3. Verfahren nach Anspruch 2, dadurch gekennzeichnet, daß das Monosaccharid D-Glucose ist.

4. Verfahren nach Anspruch 2, dadurch gekennzeichnet, daß das Monosaccharid eine Amino-desoxy-hexose ist.

5. Verfahren nach Anspruch 1, dadurch gekennzeichnet, daß das Saccharid ein Disaccharid ist, das unter Cellobiose, Lactose und Maltose ausgewählt ist.

6. Verfahren zur Herstellung von Alkyl-1-thioglykosiden nach Anspruch 1, dadurch gekennzeichnet, daß das molare Verhältnis zwischen dem Thiol und dem Saccharid etwa 1 bis 2 beträgt.

7. Verfahren zur Herstellung von Alkyl-1-thioglykosiden nach Anspruch 6, dadurch gekennzeichnet, daß das molare Verhältnis zwischen dem Thiol und dem Saccharid bei 1 liegt.

8. Alkyl-1-thioglykoside hergestellt nach dem Verfahren nach Anspruch 1, dadurch gekennzeichnet, daß sie in der Form ihres Anomerengemisches α und β vorliegen und eine lange Alkylkette oder Fettkette aufweisen.

9. Verwendung von Alkyl-1-thioglykosiden nach Anspruch 8 als nicht-ionische Detergenzien, insbesondere zur Solubilisation, Extraktion und Rekonstitution von Zellwandproteinen.

## Claims

1. Process for the preparation of alkyl-1-thioglycosides with an aliphatic chain from a saccharide chosen from among the monosaccharides and reducing di- and oligo-saccharides, characterized in that the said saccharide is reacted with a thiol in the reactive solvent pyridinium poly-(hydrogen fluoride), the number of carbon atoms of the said thiol being between 5 and 20.

2. Process according to Claim 1, characterized in that the said saccharide is a monosaccharide chosen from among the hexoses and pentoses.

3. Process according to Claim 2, characterized in that the said monosaccharide is D-glucose.

4. Process according to Claim 2, characterized in that the said monosaccharide is an amino-deoxy-hexose.

5. Process according to Claim 1, characterized in that the said saccharide is a disaccharide chosen from among cellobiose, lactose and maltose.

6. Process for preparing alkyl-1-thioglycosides according to Claim 1, characterized in that the molar ratio between the said thiol and the said saccharide lies between approximately 1 and 2.

7. Process for preparing alkyl-1-thioglycosides according to Claim 6, characterized in that the said molar ratio between the thiol and the saccharide is approximately equal to 1.

8. Alkyl-1-thioglycosides prepared according to the process of Claim 1 characterized in that they exist in the form of a mixture of their α and β anomers and that they have a long alkyl or aliphatic chain.

9. Application for alkyl-1-thioglycosides according to Claim 8 as non-ionic detergents, in particular for the solubilization, extraction and reconstitution of membranous proteins.